Europäisches Patentamt

European Patent Office

Office européen des brevets

(19)

(11)  EP 0 833 610 B1

(12)  FASCICULE DE BREVET EUROPEEN

(45) Date de publication et mention
de la délivrance du brevet:
**23.06.2004 Bulletin 2004/26**

(21) Numéro de dépôt: **97919442.0**

(22) Date de dépôt: **28.03.1997**

(51) Int Cl.⁷: **A61K 9/02**

(86) Numéro de dépôt international:
**PCT/FR1997/000567**

(87) Numéro de publication internationale:
**WO 1997/036575 (09.10.1997 Gazette 1997/43)**

(54) **COMPOSITIONS LAXATIVES ET LEUR PROCEDE DE FABRICATION**

ABFÜHRMITTEL UND VERFAHREN ZU DEREN HERSTELLUNG

LAXATIVE COMPOSITIONS AND METHOD FOR MAKING SAME

(84) Etats contractants désignés:
**AT BE CH DE DK ES FI FR GB GR IE IT LI LU MC
NL PT SE**

(30) Priorité: **01.04.1996 FR 9604041**

(43) Date de publication de la demande:
**08.04.1998 Bulletin 1998/15**

(73) Titulaire: **TECHNIPHARMA**
**98014 Monaco Cédex (MC)**

(72) Inventeur: **NOTE-SIMONNARD, Axelle**
**MC-98014 Monaco Cédex (MC)**

(74) Mandataire: **Burtin, Jean-François et al
Cabinet GEFIB,
55, rue Aristide Briand
92300 Levallois-Perret Cedex (FR)**

(56) Documents cités:
EP-A- 0 423 015          FR-A- 2 105 111
FR-M- 5 391              GB-A- 1 212 704

• CHEMICAL & PHARMACEUTICAL BULLETIN,
vol. 41, no. 2, Février 1993, TOKYO (JP), pages
351-356, XP000354420 T. HAKATA ET AL.:
"EFFECTS OF BASES AND ADDITIVES ON
RELEASE OF CARBON DIOXIDE FROM
EFFERVESCENT SUPPOSITORIES"
• CHEMICAL & PHARMACEUTICAL BULLETIN,
vol. 41, no. 2, Février 1993, TOKYO (JP), pages
346-351, XP000354419 T. HAKATA ET AL.:
"METHODS OF EVALUATION OF RELEASE OF
CARBON DIOXIDE FROM EFFERVESCENT
SUPPOSITORIES"
• DATABASE WPI Week 8551 Derwent
Publications Ltd., London, GB; AN 85-320778
XP002021616 & JP 60 224 614 A (KAO CORP) , 9
Novembre 1985

EP 0 833 610 B1

**Description**

La présente invention se rapporte au domaine de la chimie thérapeutique et plus particulièrement à celui de la pharmacotechnie.

Elle a plus précisément pour objet de nouvelles compositions pharmaceutiques à propriétés laxatives dont la caractéristique principale est de renfermer un mélange effervescent dégageant du gaz carbonique d'une manière rapide et progressive.

L'invention se rapporte spécifiquement à de nouvelles compositions pharmaceutiques destinées à la voie rectale susceptibles de provoquer par réaction chimique des principes actifs au contact de l'humidité présente dans l'ampoule rectale un dégagement gazeux important et rapide.

Le brevet français n° 788.198 (Waldenmeyer J.G.) a déjà décrit un procédé de réalisation de suppositoires effervescents qui offre la possibilité de faire dégager du gaz carbonique à l'état naissant sous l'action de l'humidité, de la chaleur ou de toute autre cause et dans lesquels les matières premières dégageant par leur mélange cet acide sont enrobées à titre de protection dans une matière grasse qui évite ainsi une réaction chimique prématurée et protège de ce fait contre une décomposition. Ces suppositoires cependant contiennent dans leur masse des ingrédients hydrophiles qui permettent aux fluides biologiques de pénétrer dans la masse et de permettre le démarrage de la réaction chimique qui provoque l'effet physiologique.

Par la suite la demande de brevet français n° 94.11913 déposée le 5 octobre 1994 au nom de la demanderesse a décrit un nouveau procédé pour fabriquer de tels suppositoires effervescents à base de tartrate acide de potassium et de bicarbonate de sodium, stables à la conservation.

Les documents (i) CHEMICAL & PHARMACEUTICAL BULLETIN, vol. 41, no. 2, Février 1993, TOKYO (JP), pages 351-356, XP000354420 T. HAKATA ET AL.: 'EFFECTS OF BASES AND ADDITIVES ON RELEASE OF CARBON DIOXIDE FROM EFFERVESCENT SUPPOSITORIES' ; (ii) CHEMICAL & PHARMACEUTICAL BULLETIN, vol. 41, no. 2, Février 1993, TOKYO (JP), pages 346-351, XP000354419 T. HAKATA ET AL.: 'METHODS OF EVALUATION OF RELEASE OF CARBON DIOXIDE FROM EFFERVESCENT SUPPOSITORIES' et (iii) FR-A-5 391 décrivent des suppositoires effervescents qui contiennent bicarbonate de sodium et phosphate monosodique.

GB-A-1 212 704 décrit des suppositoires vaginaux comprenant du bicarbonate de sodium, du citrate monosodique et d'un diluant ou véhicule adapté à la réalisation d'une forme pharmaceutique destinée à la voie rectale, notamment la suppocire Gattefosse.

DATABASE WPI Week 8551 Derwent Publications Ltd., London, GB; AN 85-320778 XP002021616 & JP 60 224 614 A (KAO CORP) du 9 Novembre 1985 décrit des suppositoires qui comprennent un bicarbonate de métal alcalin et un composé à réaction acide parmi lesquels on trouve le citrate de sodium, l'acide glutamique le phosphate monosodique et le phosphate monopotassique.

Le problème qui s'est posé pour la réalisation des suppositoires décrits dans la littérature antérieure a été de pouvoir former une masse suffisamment effervescente, c'est-à-dire dégageant un volume de gaz carbonique suffisamment important en un laps de temps suffisamment court pour obtenir une distension marquée des parois de l'ampoule rectale, qui amène un réflexe exonérateur.

Il existe différentes solutions pour tenter de résoudre ce problème. La première solution consiste à augmenter la quantité de principes actifs réactifs pour accroître le volume de gaz carbonique dégagé. Cependant la taille d'un suppositoire atteint rapidement une limite et de ce fait il n'est pas possible d'y incorporer davantage d'ingrédients actifs sans mettre en cause la conservation et l'homogénéité des suppositoires.

L'autre solution consiste à incorporer à la masse de la composition pharmaceutique destinée à la voie rectale, un autre constituant acide plus réactif que le tartrate acide de potassium et présentant toutes les garanties nécessaires de tolérance locale et l'absence de risque toxicologique dont la limite reste dans la nécessité de ne pas avoir un dégagement de $CO_2$ trop brutal.

Cette solution a servi de base à l'invention objet de la présente demande de brevet.

L'invention consiste donc en un procédé de réalisation des compositions pharmaceutiques destinées à la voie rectale tell que décrit dans la recendication 1.

La formulation effervescente impose la présence d'un élément alcalin susceptible de dégager facilement du gaz carbonique par réaction chimique avec un composé acide. L'expérience montre qu'avec des carbonates alcalins, alcalino-terreux ou d'autres métaux la réaction est beaucoup plus lente et rarement complète dans un laps de temps relativement court. Par ailleurs le choix du composé acide nécessite le respect de deux conditions. Il ne faut pas que ce composé acide soit irritant ou toxique d'une part, et qu'il ne déclenche pas une réaction d'effervescence trop violente ou trop rapide au contact de l'agent alcalin. Il n'y a qu'un nombre limité de composés acides qui satisfont à ces deux conditions. Parmi les facteurs qui peuvent intervenir pour le respect de ces deux conditions il y a en premier lieu le pka du composé acide utilisé et d'autre part la solubilité dans l'eau ou les milieux biologiques du composé acide. C'est ainsi qu'un composé comme le phtalimide benzoïque ou le succinimide du fait de leur faible solubilité dans l'eau n'amène qu'un dégagement limité et retardé de gaz carbonique. Il est possible également que leur faible degré d'acidité joue un certain rôle.

Inversement l'incorporation dans le mélange de composés acides forts ou d'acides très solubles dans les milieux aqueux conduit à un dégagement gazeux très violent ou trop rapide qui exclut l'utilisation de tels composés.

Selon les besoins, la teneur en composé acide sera celle qui correspond à peu près également sur le plan molaire à la formulation définie dans la demande de brevet antérieure en France n° 94.11913 au nom de la Demanderesse à savoir 1,150 g de tartrate acide de potassium pour 0,700 g de bicarbonate de sodium par suppositoire adulte, c'est-à-dire entre 1,15 g et 1,20 g de préférence 1,184 g pour le phosphate monosodique, entre 1,25 et 1,30 g et de préférence 1,2847 g pour le citrate monosodique, ou, pour une substance diacide comme l'acide glutamique, entre 0,85 et 0,90 g et de préférence 0,878 g et pour l'acide pyroglutamique entre 0,85 et 0,90 g et de préférence 0,889 g. Dans ces conditions le mélange est susceptible de dégager un volume de $CO_2$ mesuré au calcimètre de Bernard, à la pression atmosphérique, de l'ordre de 100 à 120 ml pour un suppositoire en l'espace de 20 minutes.

L'étude du dégagement de gaz carbonique montre que dans les 5 premières minutes il se produit un dégagement gazeux maximal qui représente environ 80 % du dégagement total. Le dégagement se ralentit ensuite pour atteindre une valeur asymptotique au bout de 10 minutes, et au bout de 20 minutes les variations de volume deviennent très faibles. Selon la nature de l'agent acide, ce dégagement gazeux peut être très rapide et presque total au bout de 5 minutes ou bien au contraire rester faible et nécessiter plus de 40 minutes pour atteindre son achèvement. C'est donc entre ces deux valeurs extrêmes que se situe l'optimum thérapeutique.

L'excipient hydrophile est une lécithine végétale ou animale, comme par exemple la lécithine de soja ou les lécithines de jaune d'oeuf.

L'agent opacifiant est un produit minéral pulvérulent, insoluble dans l'excipient hydrophile et dans l'excipient gras. De préférence l'agent opacifiant sera un silicate d'aluminium, un silicate de magnésium, un oxyde de titane ou une silice.

L'excipient gras est une graisse à point de fusion située vers 40°C comme le beurre de cacao, le beurre de karité, le beurre d'illipé ou des mélanges de ceux-ci ou bien un stéarate de polyalcools comme un stéarate de glycéryle ou de polyéthylèneglycol semi-synthétique ou synthétique, dont le point de fusion se situe dans cette gamme de températures comme les stéarates de polyéthylèneglycol commercialisés sous la dénomination Labrafil et les glycérides semi-synthétiques décrits à la pharmacopée française 10[e] Edition et commercialisés par le nom d'Estaram, Suppocire...

Dans le cas de capsules rectales on n'utilise pas d'excipient gras ou on ne l'utilise qu'en petites quantités pour assurer une masse solide homogène. Cette préparation est introduite dans une capsule de gélatine dont l'épaisseur de paroi varie de 1 à 3 mm.

Les exemples suivants illustrent les principales caractéristiques de l'invention sans toutefois les limiter.

Mesure de l'effervescence par dégagement de $CO_2$

Fonctionnement du calcimètre de Bernard.

L'essai consiste à vérifier le fonctionnement de l'installation du calcimètre de Bernard.

Matériel :

* Une ampoule de 500 ml
* Un tube gradué jusqu'à 200 ml
* Un tube en verre
* Un ballon à fond plat de 250 ml, à tubulure latérale inclinée à 30°C
* Un bouchon en caoutchouc perforé d'un trou pour le col du ballon
* Un bouchon en caoutchouc plein pour la tubulure latérale portant une nacelle pour poudres et suppositoires
* Un agitateur magnétique chauffant
* Solution saturée de chlorure de sodium

Modification :

On a apporté une légère modification sur le bouchon de la tubulure latérale par l'ajout d'un robinet. Cet ajout est nécessaire pour qu'au temps 0, l'expérience démarre avec une pression intérieure, égale à la pression atmosphérique.

Installation :

* Fixer l'ampoule et le tube gradué à un support
* Relier l'ampoule et le bas du tube gradué par un tuyau permettant la communication de la solution saturée de chlorure de Na entre les deux installations
* Relier le haut du tube gradué et le ballon de réaction par un tube en verre à travers lequel remonte le $CO_2$ en provenance du ballon

Mode opératoire :

* Introduire une solution saturée de chlorure de sodium dans l'ampoule et le tube gradué
* Ajuster le niveau du tube gradué avant de faire démarrer l'expérience
* Poser sur l'agitateur magnétique le ballon contenant 100 ml d'eau distillée à 37° C
* Boucher le ballon avec un bouchon fixé préalablement au bout du tube de dégagement de $CO_2$
* Introduire le suppositoire dans le ballon sans qu'il y ait contact entre le suppositoire et l'eau
* Vérifier à nouveau le niveau du chlorure de Na dans le tube gradué avant de fermer le robinet du bouchon latéral
* Fermer le robinet
* Mettre l'agitateur en marche et laisser tomber le suppositoire, sans oublier de déclencher le chronomètre
* L'expérience se déroule à la température du laboratoire (environ 20°C).

Intensité de la réaction entre les composants de base

Cet essai effectué sur les deux principes actifs de base respectant les quantités de chaque produit selon la formule de l'industrie.

Mode opératoire :

| Bicarbonate de Na | 0,700 g |
|---|---|
| Tartrate acide de potassium | 1,150 g |

Peser exactement les quantités nécessaires des deux principes actifs pour un suppositoire. Mélanger à la main et effectuer l'expérience.

| ESSAI |
|-------|
| 1 |

| Temps (minute) | ml |
|----------------|------|
| 1 | 2,5 |
| 2 | 49,2 |
| 3 | 66,8 |
| 4 | 73 |
| 5 | 83,2 |
| 6 | 94,6 |
| 7 | > 100 |

Fabrication des suppositoires

On a fabriqué 10 lots de suppositoires selon le protocole de l'industrie et on les a testés le même jour.

Les suppositoires restent insolubles à la température du laboratoire (20°C) lors de l'étude du dégagement de $CO_2$

Il faut donc chauffer l'eau jusqu'à 37°C. On a donc utilisé un agitateur magnétique à plaque chauffante.

On a refait la courbe de référence de deux principes actifs à 37° C

- Proportions suppositoires adultes :

  - Bicarbonate de $N_a$ 0,700 g
  - Tartrate acide de K 1,150 g

nbre d'essais $\boxed{5}$

| Temps | ESSAI 1 ml | ESSAI 2 ml | ESSAI 3 ml | ESSAI 4 ml | ESSAI 5 ml | MOYENNE ml |
|---|---|---|---|---|---|---|
| 1 | 44,2 | 43 | 42,6 | 44,2 | 44,8 | 43,76 |
| 2 | 57,8 | 56,2 | 50 | 62 | 61,3 | 57,46 |
| 3 | 67,2 | 65,8 | 63,4 | 72,6 | 70,8 | 67,96 |
| 4 | 73,8 | 73,2 | 72,2 | 79,1 | 77,2 | 75,1 |
| 5 | 78,8 | 78,6 | 78,5 | 84,2 | 81,8 | 80,38 |
| 6 | 82,7 | 82,6 | 83,4 | 87,8 | 85,4 | 84,38 |
| 7 | 85,8 | 85,9 | 87,3 | 90,6 | 88,2 | 87,56 |
| 8 | 88 | 88,5 | 90,2 | 92,9 | 90,2 | 89,96 |
| 9 | 91,2 | 90,8 | 92,3 | 94,6 | 92,2 | 92,22 |
| 10 | 92,2 | 92,6 | 94 | 96,2 | 93,4 | 93,68 |
| 11 | 94 | 94,1 | 95,1 | 97,4 | 94,6 | 95,04 |
| 12 | 95,2 | 95,2 | 96,2 | 98,4 | 95,6 | 96,12 |
| 13 | 96,2 | 96,2 | 96,8 | 99 | 96,2 | 96,88 |
| 14 | 96,7 | 97 | 97,6 | 99,6 | 96,7 | 97,52 |
| 15 | 97,6 | 97,8 | 98,4 | 100 | 97,2 | 98,2 |
| 16 | 98,4 | 98,4 | 99 | 100 | 97,8 | 98,72 |
| 17 | 99 | 99 | 99,4 | 100 | 98,1 | 99,1 |
| 18 | 99,6 | 99,5 | 99,7 | 100 | 98,4 | 99,44 |
| 19 | 100 | 99,8 | 99,8 | 100 | 98,4 | 99,6 |
| 20 | 100 | 100 | 99,8 | 100 | 98,4 | 99,64 |
| 21 | | | | | | |

- Proportions suppositoires enfants :

  • Bicarbonate de $N_a$ 0,350 g
  • Tartrate acide de K 0,575 g

nombre d'essais

| 3 |

| | ESSAI 2 | ESSAI 3 | ESSAI 4 | MOYENNE |
|---|---|---|---|---|
| Temps | ml | ml | ml | ml |
| 1 | 16,2 | 15,4 | 14,4 | 15,33 |
| 2 | 25,8 | 25,4 | 24,6 | 25,27 |
| 3 | 32,8 | 32,8 | 32,1 | 32,57 |
| 4 | 37,9 | 38,2 | 37,8 | 37,97 |
| 5 | 42 | 42,2 | 41,8 | 42,00 |
| 6 | 45,1 | 45 | 44,8 | 44,97 |
| 7 | 47,2 | 47,4 | 47,2 | 47,27 |
| 8 | 48,8 | 49 | 48,9 | 48,90 |
| 9 | 49,9 | 50,4 | 50,1 | 50,13 |
| 10 | 50,6 | 51,6 | 51 | 51,07 |
| 11 | 51,4 | 52,7 | 51,9 | 52,00 |
| 12 | 52 | 53,3 | 52,6 | 52,63 |
| 13 | 52,4 | 53,6 | 53 | 53,00 |
| 14 | 52,6 | 54 | 53,4 | 53,33 |
| 15 | 53 | 54,4 | 53,8 | 53,73 |
| 16 | 53 | 54,6 | 54 | 53,87 |
| 17 | 52,3 | 54,9 | 54,2 | 53,80 |
| 18 | 54 | 55 | 54,2 | 54,40 |
| 19 | 54,2 | 55 | 54,2 | 54,47 |
| 20 | 54,4 | 55 | 54,2 | 54,53 |

## I - ETUDE DE FORMULATION SELON L'INVENTION SACCHARINE ACIDE

Cet essai commence par le calcul stoechiométrique pour déterminer la quantité de saccharine nécessaire à utiliser sans changer la quantité de bicarbonate contenu dans la formule industrielle.

$$\text{Saccharine } (C_7H_5O_3NS) ; 1 \text{ mole} = 183g \rightarrow 6{,}10^{-3} \text{ mole} = 1{,}119g$$

Pour un suppo adulte il faut 1,119g de saccharine acide.
Pour un suppo enfant il faut 1,119g : 2 = 0,5595g de saccharine acide

Formule :

| Saccharine acide | 0,5595g |
|---|---|
| Bicarbonate Na | 0,350g |

Tamiser la saccharine avant la pesée (tamis 0,315)

Résultats de dégagement:

| nbre d'essais | | 2 | |
|---|---|---|---|
| | ESSAI | ESSAI | MOYENNE |
| | 0.5595 g | 0.5595 g | |
| Temps | ml | ml | ml |
| 1 | 24,00 | 27,60 | 25,80 |
| 2 | 28,40 | 33,40 | 30,90 |
| 3 | 32,10 | 37,50 | 34,80 |
| 4 | 35,00 | 40,30 | 37,65 |
| 5 | 37,40 | 42,60 | 40,00 |
| 6 | 39,40 | 44,40 | 41,90 |
| 7 | 41,30 | 45,80 | 43,55 |
| 8 | 42,90 | 47,00 | 44,95 |
| 9 | 44,20 | 48,00 | 46,10 |
| 10 | 45,40 | 48,80 | 47,10 |
| 11 | 46,50 | 49,40 | 47,95 |
| 12 | 47,40 | 50,20 | 48,80 |
| 13 | 48,10 | 50,80 | 49,45 |
| 14 | 48,80 | 51,30 | 50,05 |
| 15 | 49,40 | 51,50 | 50,45 |
| 16 | 50,00 | 51,80 | 50,90 |
| 17 | 50,40 | 51,90 | 51,15 |
| 18 | 50,80 | 52,00 | 51,40 |
| 19 | 51,20 | 52,20 | 51,70 |
| 20 | 51,60 | 52,40 | 52,00 |
| 21 | 51,80 | 52,60 | 52,20 |

**II - ACIDE PYROGLUTAMIQUE**

Calcul stoechiométrique :

Acide pyroglutamique ($C_5H_7O_3N$) ; 1 mole = 147g $\rightarrow$ $6,10^{-3}$ mole = 0,899g

Pour un suppo adulte il faut 0,899g d'acide pyroglutamique

Pour un suppo enfant il faut 0,899g : 2 = 0,4495g d'acide pyroglutamique

Formule :

| Acide pyroglutamique | 0,4495g |
|---|---|
| Bicarbonate Na | 0,350g |

Réduction de la quantité d'acide pyroglutamique

Formule :

| Acide pyroglutamique | 0,400g |
|---|---|
| Bicarbonate Na | 0,350g |

Conclusion :

Avec l'acide pyroglutamique (0,4495g) le même phénomène se produit qu'avec la saccharine acide : dans les 2 premières minutes le dégagement de $CO_2$ est important.
Avec un taux d'acide pyroglutamique un peu plus faible (0,400g), le temps de dégagement de $CO_2$ est un peu plus court.

**III - PHOSPHATE MONOPOTASSIQUE CRIST.**

Calcul stoechiométrique :

$$\text{Phosphate monopotassique crist.}(H_2KO_4P) \; ; \; 1 \text{ mole} = 136,09g \rightarrow$$

$$6,10^{-3} \text{ mole} = 0,832g$$

Pour un suppo adulte il faut 0,832g de phosphate monopotassique
Pour un suppo enfant il faut 0,832g : 2 = 0,416g phosphate monopotassique

Formule :

| Phosphate monopotassique | 0,416g |
|---|---|
| Bicarbonate Na | 0,350g |

Conclusion :

Dégagement faible de $CO_2$.
Utiliser une quantité double par rapport au calcul stoechiométrique.

Résultats de dégagement :

nbre d'essais 3

| Dose de phosphate monopotassique | Bicar 0,7 g | | | |
|---|---|---|---|---|
| | ESSAI | ESSAI | ESSAI | MOYENNE |
| | 0,832 g | 0,832 g | 0,832 g | |
| Temps | ml | ml | ml | ml |
| 1 | 15,2 | 13 | 12,8 | 13,67 |
| 2 | 22 | 19,8 | 20,4 | 20,73 |
| 3 | 27,6 | 25,4 | 26,5 | 26,50 |
| 4 | 32,6 | 30,1 | 31,8 | 31,50 |
| 5 | 36,9 | 34,2 | 36,2 | 35,77 |
| 6 | 40,6 | 37,7 | 40 | 39,43 |
| 7 | 43,9 | 40,9 | 43,4 | 42,73 |
| 8 | 46,8 | 43,5 | 46,1 | 45,47 |
| 9 | 49,1 | 45,8 | 48,6 | 47,83 |
| 10 | 51,4 | 48 | 50,8 | 50,07 |
| 11 | 53,3 | 49,8 | 52,7 | 51,93 |
| 12 | 54,8 | 51,3 | 54,2 | 53,43 |
| 13 | 56,2 | 52,7 | 55,7 | 54,87 |
| 14 | 57,4 | 54,1 | 56,9 | 56,13 |
| 15 | 58,5 | 55,4 | 58 | 57,30 |
| 16 | 59,6 | 56,5 | 58,8 | 58,30 |
| 17 | 60,4 | 57,4 | 59,7 | 59,17 |
| 18 | 61,1 | 58,4 | 60,5 | 60,00 |
| 19 | 61,8 | 59,2 | 61,1 | 60,70 |
| 20 | 62,4 | 59,8 | 61,6 | 61,27 |
| 21 | 63 | 60,3 | 62,1 | 61,80 |
| 22 | 63,4 | 60,9 | 62,6 | 62,30 |
| 23 | 63,8 | 61,4 | 63,1 | 62,77 |
| 24 | 64,3 | 62 | 63,4 | 63,23 |
| 25 | 64,7 | 62,4 | 63,8 | 63,63 |
| 26 | 65 | 62,6 | 64 | 63,87 |
| 27 | 65,2 | 62,8 | 64,3 | 64,10 |
| 28 | 65,4 | 63 | 64,5 | 64,30 |
| 29 | 65,5 | 63,2 | 64,5 | 64,40 |
| 30 | 65,6 | 63,2 | 64,5 | 64,43 |
| 31 | 65,8 | 63,2 | 64,5 | 64,50 |

**IV - PHOSPHATE MONOSODIQUE ANHYDRE**

Calcul stoechiométrique :

$$\text{Phosphate monosodique anhydre } (NaH_2PO_4) \text{ ; 1 mole} = 119{,}98g \rightarrow$$

$$6{,}10^{-3} \text{ mole} = 0{,}734g$$

Pour un suppo adulte il faut 0,734g phosphate monosodique
Pour un suppo enfant il faut 0,734g : 2 = 0,366g phosphate monosodique
Formule : quantité double par rapport au calcul stoechiométrique.

| Phosphate monosodique | 0,734g |
|---|---|
| Bicarbonate Na | 0,700g |

Résultats de dégagement :

nbre d'essais     3

Bicar:0,7 g. Tamis 0,250 mm

| Phosphate monosodique | ESSAI 0,734 g | ESSAI 0,734 g | ESSAI 0,734 g | MOYENNE |
|---|---|---|---|---|
| Temps | ml | ml | ml | ml |
|  |  |  |  |  |
| 1 | 16,2 | 16,5 | 13,6 | 15,43 |
| 2 | 24,2 | 24,9 | 21,2 | 23,43 |
| 3 | 30,4 | 31,8 | 28 | 30,07 |
| 4 | 35,5 | 37,3 | 33,8 | 35,53 |
| 5 | 39,8 | 41,9 | 38,6 | 40,10 |
| 6 | 43,3 | 45,5 | 42,2 | 43,67 |
| 7 | 46,2 | 48,8 | 45,4 | 46,80 |
| 8 | 48,7 | 51,3 | 48,1 | 49,37 |
| 9 | 50,9 | 53,6 | 50,4 | 51,63 |
| 10 | 52,7 | 55,5 | 52,4 | 53,53 |
| 11 | 54,2 | 57,2 | 54,1 | 55,17 |
| 12 | 55,6 | 58,6 | 55,6 | 56,60 |
| 13 | 56,9 | 59,7 | 57 | 57,87 |
| 14 | 58,2 | 60,9 | 58 | 59,03 |
| 15 | 59,2 | 61,8 | 59 | 60,00 |
| 16 | 60,2 | 62,4 | 59,6 | 60,73 |
| 17 | 61 | 63 | 60,3 | 61,43 |
| 18 | 61,6 | 63,4 | 60,8 | 61,93 |
| 19 | 62,2 | 63,8 | 61,2 | 62,40 |
| 20 | 62,6 | 64,1 | 61,6 | 62,77 |
| 21 | 63,1 | 64,4 | 61,9 | 63,13 |
| 22 | 63,4 | 64,6 | 62,1 | 63,37 |
| 23 | 63,8 | 64,8 | 62,3 | 63,63 |
| 24 | 64,2 | 64,9 | 62,5 | 63,87 |
| 25 | 64,5 | 64,9 | 62,6 | 64,00 |
| 26 | 64,6 | 64,9 | 62,8 | 64,10 |
| 27 | 64,8 | 64,9 | 62,8 | 64,17 |
| 28 | 64,9 | 64,9 | 62,8 | 64,20 |
| 29 | 65 | 64,9 | 62,8 | 64,23 |
| 30 |  |  |  |  |

**V - SUCCINIMIDE**

Calcul stoechiométrique :

$$\text{Succinimide } (C_4H_5NO_2) \; ; \; 1 \text{ mole} = 99{,}09g \rightarrow 6{,}10^{-3} \text{ mole} = 0{,}606g$$

Pour un suppo adulte il faut 0,606g de Succinimide
Pour un suppo enfant il faut 0,606g : 2 = 0,303g Succinimide

Formule :

| Succinimide | 0,303g |
|---|---|
| Bicarbonate Na | 0,305g |

Résultats de dégagement : (voir tableau)

Conclusion :

Le dégagement de $CO_2$ est dérisoire en comparaison avec les quantités de principe actif prévues par le calcul stoechiométrique.

| ESSAI 0,303 g | nbre d'essais 1 |
|---|---|
| **Temps** | **ml** |
| 1 | 2,0 |
| 2 | 2,6 |
| 3 | 3,1 |
| 4 | 3,4 |
| 5 | 3,7 |
| 6 | 4,1 |
| 7 | 4,4 |
| 8 | 4,7 |
| 9 | 5,1 |
| 10 | 5,3 |
| 11 | 5,6 |
| 12 | 5,9 |
| 13 | 6,1 |
| 14 | 6,3 |
| 15 | 6,6 |
| 16 | 6,8 |
| 17 | 7,0 |
| 18 | 7,3 |
| 19 | 7,5 |
| 20 | 7,6 |
| 21 | 7,8 |
| 22 | 7,9 |
| 23 | 8,0 |
| 24 | 8,1 |
| 25 | 8,2 |
| 26 | 8,3 |

B - ESSAI GALENIQUE DE SUPPOSITOIRES AVEC LE PHOSPHATE MONOSODIQUE ANHYDRE

Pour avoir un bon dégagement de $CO_2$ on a été obligé de mettre le double de la quantité stoechiométrique.

Formule de référence pour enfants :

| Tartrate acide de K | 0,575g |
|---|---|
| Bicarbonate de Na | 0,350g |
| Lécithine de soja | 0,105g |
| Talc | 0,0525g |
| Suppocire | 0,9175g |
| | 2,000g |

Utilisation phosphate monosodique anhydre à la place de tartrate acide de K :

Formule :

| Phosphate monosodique anhydre | 0,734g |
|---|---|
| Bicarbonate de Na | 0,700g |
| Lécithine de soja | 0,105g |
| Talc | 0,0525g |
| Suppocire | 0,4085g |
| | 2,000g |

Calculé pour 10 suppos.

Résultats :

La fabrication des suppos s'est avérée difficile car il a fallu chauffer jusqu'à 60°C pour remplir les alvéoles d'une part et la masse était trop pâteuse et granuleuse d'autre part. Cette difficulté est peut être due à la faible quantité de suppocire utilisée.
Le test de dégagement de $CO_2$ effectué après 24 heures est indiqué dans le tableau ci-après.

| Temps | ESSAI 2,3738g ml | ESSAI 2,3687g ml | ESSAI 2,4g ml | MOYENNE 2,38083g ml | CORRECTION 2g |
|---|---|---|---|---|---|
| 1 | 4,5 | 6 | 6,2 | 5,57 | 4,68 |
| 2 | 23,4 | 23,2 | 26,6 | 24,40 | 20,50 |
| 3 | 34,8 | 34,4 | 40,2 | 36,47 | 30,63 |
| 4 | 43,4 | 42,2 | 49,4 | 45,00 | 37,80 |
| 5 | 49,8 | 48,5 | 56,1 | 51,47 | 43,23 |
| 6 | 54,6 | 52,3 | 61,1 | 56,00 | 47,04 |
| 7 | 58,3 | 55,2 | 64,8 | 59,43 | 49,93 |
| 8 | 61,1 | 58,2 | 67,8 | 62,37 | 52,39 |
| 9 | 63,2 | 60,7 | 69,8 | 64,57 | 54,24 |
| 10 | 65 | 63 | 71,4 | 66,47 | 55,84 |
| 11 | 66,3 | 64,8 | 72,6 | 67,90 | 57,04 |
| 12 | 67,5 | 66,1 | 73,6 | 69,07 | 58,02 |
| 13 | 68,4 | 67,2 | 74,1 | 69,90 | 58,72 |
| 14 | 69,1 | 68,4 | 74,8 | 70,77 | 59,45 |
| 15 | 69,8 | 69,4 | 75,4 | 71,53 | 60,09 |
| 16 | 70,4 | 70,3 | 75,8 | 72,17 | 60,62 |
| 17 | 71 | 71,4 | 76,2 | 72,87 | 61,21 |
| 18 | 71,6 | 72,2 | 76,4 | 73,40 | 61,66 |
| 19 | 72 | 72,8 | 76,8 | 73,87 | 62,05 |
| 20 | 72,4 | 73,3 | 77 | 74,23 | 62,36 |
| 21 | 72,7 | 73,6 | 77,2 | 74,50 | 62,58 |
| 22 | 72,8 | 74 | 77,2 | 74,67 | 62,72 |
| 23 | 72,9 | 74,3 | 77,2 | 74,80 | 62,84 |
| 24 | 73,2 | 74,7 | 77,2 | 75,03 | 63,03 |
| 25 | 73,2 | 75 | 77,2 | 75,13 | 63,12 |
| 26 | 73,2 | 75,4 | 77,2 | 75,27 | 63,23 |
| 27 | 73,2 | 75,5 | 77,2 | 75,30 | 63,26 |
| 28 | 73,2 | 75,7 | 77,2 | 75,37 | 63,31 |

Conclusion :

La comparaison des courbes obtenues avec les poudres de tartrate plus bicarbonate Na d'une part et les poudres $PO_4HNa$ plus bicarbonate Na d'autre part avec la courbe des suppositoires de $PO_4HNa$ plus bicarbonate Na montre qu'il n'y a pas de réaction entre la poudre et les excipients. Les suppositoires donnent une courbe identique à celle du mélange des poudres seules.

nbre d'essais | 3 |

| Temps | ESSAI TARTRE ml | ESSAI HPO4Na ml | ESSAI Suppo 2 g ml |
|---|---|---|---|
| 1 | 15,33 | 15,43 | 4,68 |
| 2 | 25,27 | 23,43 | 20,5 |
| 3 | 32,57 | 30,07 | 30,63 |
| 4 | 37,97 | 35,53 | 37,8 |
| 5 | 42,00 | 40,1 | 43,23 |
| 6 | 44,97 | 43,67 | 47,04 |
| 7 | 47,27 | 46,8 | 49,93 |
| 8 | 48,90 | 49,37 | 52,39 |
| 9 | 50,13 | 51,63 | 54,24 |
| 10 | 51,07 | 53,53 | 55,84 |
| 11 | 52,00 | 55,17 | 57,04 |
| 12 | 52,63 | 56,6 | 58,02 |
| 13 | 53,00 | 57,87 | 58,72 |
| 14 | 53,33 | 59,03 | 59,45 |
| 15 | 53,73 | 60 | 60,09 |
| 16 | 53,87 | 60,73 | 60,62 |
| 17 | 53,80 | 61,43 | 61,21 |
| 18 | 54,40 | 61,93 | 61,66 |
| 19 | 54,47 | 62,4 | 62,05 |
| 20 | 54,53 | 62,77 | 62,36 |
| 21 | 54,53 | 63,13 | 62,58 |
| 22 | | 63,37 | 62,72 |
| 23 | | 63,63 | 62,84 |
| 24 | | 63,87 | 63,03 |
| 25 | | 64 | 63,12 |
| 26 | | 64,1 | 63,23 |
| 27 | | 64,17 | 63,26 |
| 28 | | 64,2 | 63,31 |
| 29 | | 64,23 | |

## COMPARAISON DES DIFFERENTS DOSAGES DE $PO_4H_2Na$

Le tableau ci-dessous indique les résultats regroupés des différents dosages de suppositoires de phosphate monosodique anhydre par rapport aux suppositoires de 2 g à fabrication industrielle et aux suppositoires de 2 g fabriqués au Laboratoire GALENIQUE.

| | | | PO4HNab0.592 g | PO4HNa0.5624 | PO4HNa0.497 g | PO4HNa0.367 g |
|---|---|---|---|---|---|---|
| temps | moyindus2gr | moylab2gr | moylab2gr | moylab2gr | moylab2gr | moylab2gr |
| 0 | 0.00 | 0.00 | 0.00 | 0.00 | 0.00 | 0.00 |
| 1 | 2.20 | 3.48 | 5.88 | 4.79 | 3.83 | 3.63 |

(suite)

| temps | moyindus2gr | moylab2gr | PO4HNab0.592 g | PO4HNa0.5624 | PO4HNa0.497 g | PO4HNa0.367 g |
|---|---|---|---|---|---|---|
| | | | moylab2gr | moylab2gr | moylab2gr | moylab2gr |
| 2 | 5.20 | 9.00 | 16.79 | 13.19 | 9.69 | 7.04 |
| 3 | 8.61 | 14.95 | 22.67 | 19.24 | 14.51 | 10.02 |
| 4 | 11.97 | 20.10 | 27.52 | 24.03 | 18.80 | 12.52 |
| 5 | 15.21 | 24.37 | 31.50 | 28.20 | 22.34 | 14.68 |
| 6 | 18.24 | 27.52 | 35.14 | 31.46 | 25.33 | 16.47 |
| 7 | 20.95 | 30.39 | 38.25 | 34.25 | 27.24 | 17.99 |
| 8 | 23.51 | 32.68 | 41.02 | 36.79 | 29.84 | 19.23 |
| 9 | 25.98 | 34.59 | 43.27 | 38.79 | 31.78 | 20.26 |
| 10 | 28.18 | 36.35 | 45.52 | 40.48 | 33.36 | 21.13 |
| 11 | 30.42 | 37.78 | 47.34 | 41.99 | 34.79 | 21.89 |
| 12 | 32.55 | 39.11 | 48.98 | 43.34 | 36.01 | 22.65 |
| 13 | 34.52 | 40.34 | 50.54 | 44.49 | 37.09 | 23.19 |
| 14 | 36.29 | 41.49 | 51.84 | 45.40 | 38.08 | 23.73 |
| 15 | 38.02 | 42.58 | 53.14 | 46.19 | 38.90 | 24.06 |
| 16 | 39.56 | 43.69 | 54.09 | 46.88 | 39.59 | 24.60 |
| 17 | 41.06 | 44.61 | 55.04 | 47.47 | 40.20 | 25.14 |
| 18 | 42.39 | 45.59 | 55.91 | 47.97 | 40.89 | 25.68 |
| 19 | 43.73 | 46.32 | 56.60 | 48.44 | 41.32 | 26.12 |
| 20 | 44.86 | 47.11 | 57.29 | 48.85 | 41.78 | 26.55 |
| 21 | 45.89 | 47,72 | 57.81 | 49.19 | 42.03 | 26.87 |
| 22 | 46.79 | 48.39 | 58.16 | 49.45 | 42.29 | 27.15 |
| 23 | 47.56 | 49.01 | 58.42 | 49.66 | 42.54 | 27.42 |
| 24 | 48.26 | 49.48 | 58.76 | 49.88 | 42.72 | 27.63 |
| 25 | 48.83 | 50.04 | 59.20 | 50.04 | 42.95 | 27.85 |
| 26 | 49.40 | 50.53 | 59.54 | 50.13 | 43.05 | 28.07 |
| 27 | 49.80 | 50.97 | 59.71 | 50.29 | 43.13 | 28.28 |
| 28 | 50.23 | 51.40 | 59.89 | 50.39 | 43.23 | 28.39 |
| 29 | 50.56 | 51.74 | 60.41 | 50.45 | 43.23 | 28.50 |
| 30 | 50.83 | 52.10 | 60.75 | 50.45 | | 28.61 |

Etude de conservation des préparations de suppositoires en proportions adultes par vieillissement à température ambiante (20°C) dans une étuve thermostatée à hygrométrie contrôlée :

Formule de référence

Formule de référence fabriquée au Laboratoire GALENIQUE:

| Tartrate acide de potassium | 1,150 g |
|---|---|
| Bicarbonate de sodium | 0,700 g |

(suite)

| | |
|---|---|
| Lécithine de soja | 0,210 g |
| Talc | 0,105 g |
| Suppocire | 1,835 g |
| | 4,000 g |

**Résultats :**

Bonne homogénéité du lot.

| ESSAI 1 | ESSAI 2 | ESSAI 3 | MOYENNE |
|---|---|---|---|
| 3,9058 | 3,9226 | 3,9246 | 3,9177 |

| Temps | ml | ml | ml | ml |
|---|---|---|---|---|
| | | | | |
| 5 | 44,5 | 31,5 | 42 | 39,33 |
| 10 | 73 | 63 | 72 | 69,33 |
| 20 | 100,5 | 96 | 100,5 | 99,00 |
| 40 | 108,5 | 110 | 112,5 | 110,33 |

Ramené à 4 g :

| ESSAI 1 | ESSAI 2 | ESSAI 3 | MOYENNE |
|---|---|---|---|
| 4,0000 | 4,0000 | 4,0000 | 4,0000 |

| Temps | ml | ml | ml | ml |
|---|---|---|---|---|
| | | | | |
| 5 | 45,6 | 32,1 | 42,8 | 40,2 |
| 10 | 74,8 | 64,2 | 73,4 | 70,8 |
| 20 | 102,9 | 97,9 | 102,4 | 101,1 |
| 40 | 111,1 | 112,2 | 114,7 | 112,6 |

Compositions selon l'invention

1 - <u>Avec le phosphate monosodique</u>

Formule

| Phosphate monosodique | 1,184 g |
|---|---|
| Bicarbonate de sodium | 1,128 g |
| Lécithine de soja | 0,210 g |
| Talc | 0,105 g |
| Suppocire | 1,373 g |
| | 4,000 g |

Résultats :

Bonne homogénéité du lot.

| ESSAI 1 | ESSAI 2 | ESSAI 3 | MOYENNE |
|---|---|---|---|
| 4,6744 | 4,4121 | 4,3818 | 4,4894 |

| Temps | ml | ml | ml | ml |
|---|---|---|---|---|
| | | | | |
| 5 | 71,5 | 59 | 64 | 64,83 |
| 10 | 94 | 84 | 85 | 84,67 |
| 20 | 121 | 107,5 | 110 | 112,83 |
| 40 | 134 | 121,5 | 126 | 127,17 |

Ramené à 4 g :

| ESSAI 1 | ESSAI 2 | ESSAI 3 | MOYENNE |
|---|---|---|---|
| 4,0000 | 4,0000 | 4,0000 | 4,0000 |

| Temps | ml | ml | ml | ml |
|---|---|---|---|---|
| | | | | |
| 5 | 61,2 | 53,5 | 58,4 | 57,7 |
| 10 | 80,4 | 76,2 | 77,6 | 78,1 |
| 20 | 103,5 | 97,5 | 100,4 | 100,5 |
| 40 | 114,7 | 110,2 | 115,0 | 113,3 |

2 - Avec de l'acide Glutamique

Formule :

| Acide glutamique | 0,878 g |
|---|---|
| Bicarbonate de sodium | 0,700 g |
| Lécithine de soja | 0,210 g |
| Talc | 0,105 g |
| Suppocire | 2,107 g |
| | 4,000 g |

Résultats :

Lot homogène.

| ESSAI 1 | ESSAI 2 | MOYENNE |
|---|---|---|
| 3,5728 | 3,6235 | 3,5982 |

| Temps | ml | ml | ml |
|---|---|---|---|
| | | | |
| 5 | 58 | 57,5 | 57,75 |
| 10 | 85 | 81,5 | 83,25 |
| 20 | 97,5 | 100 | 98,75 |
| 40 | 101 | 102 | 101,50 |

Ramené à 4 g :

| ESSAI 1 | ESSAI 2 | MOYENNE |
|---------|---------|---------|
| 4,0000 | 4,0000 | 4,0000 |

| Temps | ml | ml | ml |
|-------|------|------|------|
|  |  |  |  |
| 5 | 64,9 | 63,5 | 64,2 |
| 10 | 95,2 | 90,0 | 92,6 |
| 20 | 109,2 | 110,4 | 109,8 |
| 40 | 113,1 | 112,6 | 112,8 |

3 - <u>Avec de l'acide pyroglutamique</u>

| Acide pyroglutamique | 0,889 g |
|----------------------|---------|
| Bicarbonate de sodium | 0,700 g |
| Lécithine de soja | 0,210 g |
| Talc | 0,105 g |
| Suppocire | 2,096 g |
|  | 4,000 g |

Résultats : Lot homogène.

| ESSAI 1 | ESSAI 2 | ESSAI 3 | MOYENNE |
|---------|---------|---------|---------|
| 3,7341 | 3,7351 | 3,5711 | 3,6801 |

| Temps | ml | ml | ml | ml |
|-------|------|------|------|------|
|  |  |  |  |  |
| 5 | 47 | 55 | 43 | 48,33 |
| 10 | 89,5 | 92,5 | 81,5 | 87,83 |
| 20 | 107,5 | 103 | 97 | 102,50 |
| 40 | 110 | 103 | 100 | 104,33 |

Ramené à 4 g :

| | ESSAI 1 | ESSAI 2 | ESSAI 3 | MOYENNE |
|---|---|---|---|---|
| | 4,0000 | 4,0000 | 4,0000 | 4,0000 |

| Temps | ml | ml | ml | ml |
|---|---|---|---|---|
| | | | | |
| 5 | 50,3 | 58,9 | 48,2 | 52,5 |
| 10 | 95,9 | 99,1 | 91,3 | 95,4 |
| 20 | 115,2 | 110,3 | 108,6 | 111,4 |
| 40 | 117,8 | 110,3 | 112,0 | 113,4 |

4 - <u>Essai avec du citrate monosodique</u>

| Formule : | |
|---|---|
| Citrate monosodique | 1,2847 g |
| Bicarbonate de sodium | 0,700 g |
| Lécithine de soja | 0,210 g |
| Talc | 0,105 g |
| Suppocire | 1,7003 g |
| | 4,000 g |

Résultats : Lot homogène.

| | ESSAI 1 | ESSAI 2 | MOYENNE |
|---|---|---|---|
| | 3,9785 | 4,148 | 4,0633 |

| Temps | ml | ml | ml |
|---|---|---|---|
| | | | |
| 5 | 98 | 110 | 104,00 |
| 10 | 124 | 131 | 127,50 |
| 20 | 142,5 | 147 | 144,75 |
| 40 | 149,5 | 149 | 149,25 |

Ramené à 4 g :

| | ESSAI 1 | ESSAI 2 | MOYENNE |
|---|---|---|---|
| | 4,0000 | 4,0000 | 4,0000 |

| Temps | ml | ml | ml |
|---|---|---|---|
| | | | |
| 5 | 98,5 | 106,1 | 102,3 |
| 10 | 124,7 | 126,3 | 125,5 |
| 20 | 143,3 | 141,8 | 142,5 |
| 40 | 150,3 | 143,7 | 147,0 |

**ETUDE COMPARATIVE DES STABILITES DES DIVERS LOTS APRES TROIS MOIS**

On a regroupé l'ensemble des courbes sur la même planche pour une visior générale des résultats.

Résultats :

*    Courbe 1 : Après vieillissement de 3 mois le dégagement de $CO_2$ a nettement augmenté.

*    Courbe 2 : Dégagement de $CO_2$ identique dans les 10 premières minutes et peu d'écart sur la fin.

*    Courbe 3 : Augmentation de dégagement de $CO_2$

*    Courbe 4 : Diminution de $CO_2$. C'est le seul des 5 produits qui a moins dégagé au bout de 3 mois de $CO_2$

*    Courbe 5 : Augmentation de dégagement de $CO_2$

## 1er Jour

| Courbe 1 | Courbe 2 | Courbe 3 | Courbe 4 | Courbe 5 |
|---|---|---|---|---|
| Sup.de réf. | Ph.monoNa | Ac.glut. | Ac.pyroglut | Cit.monoNa |

| Temps | ml | ml | ml | ml | ml |
|---|---|---|---|---|---|
|  |  |  |  |  |  |
| 5 | 26,65 | 59,4 | 41,2 | 88,9 | 70,52 |
| 10 | 52,31 | 76 | 66,6 | 107,16 | 96,99 |
| 20 | 77,31 | 93,3 | 88,5 | 122,58 | 120,97 |
| 40 | 92,93 | 99,6 | 96,7 |  | 132,51 |

## Après 3 mois

| Courbe 1 | Courbe 2 | Courbe 3 | Courbe 4 | Courbe 5 |
|---|---|---|---|---|
| Sup.de réf. | Ph.monoNa | Ac.glut. | Ac.pyroglut | Cit.monoNa |

| Temps | ml | ml | ml | ml | ml |
|---|---|---|---|---|---|
|  |  |  |  |  |  |
| 5 | 40,2 | 57,7 | 64,2 | 52,5 | 102,3 |
| 10 | 92,2 | 78,1 | 92,6 | 95,4 | 125,5 |
| 20 | 117,0 | 100,5 | 109,8 | 111,4 | 142,5 |
| 40 | 121,6 | 113,3 | 112,8 | 113,4 | 147,0 |

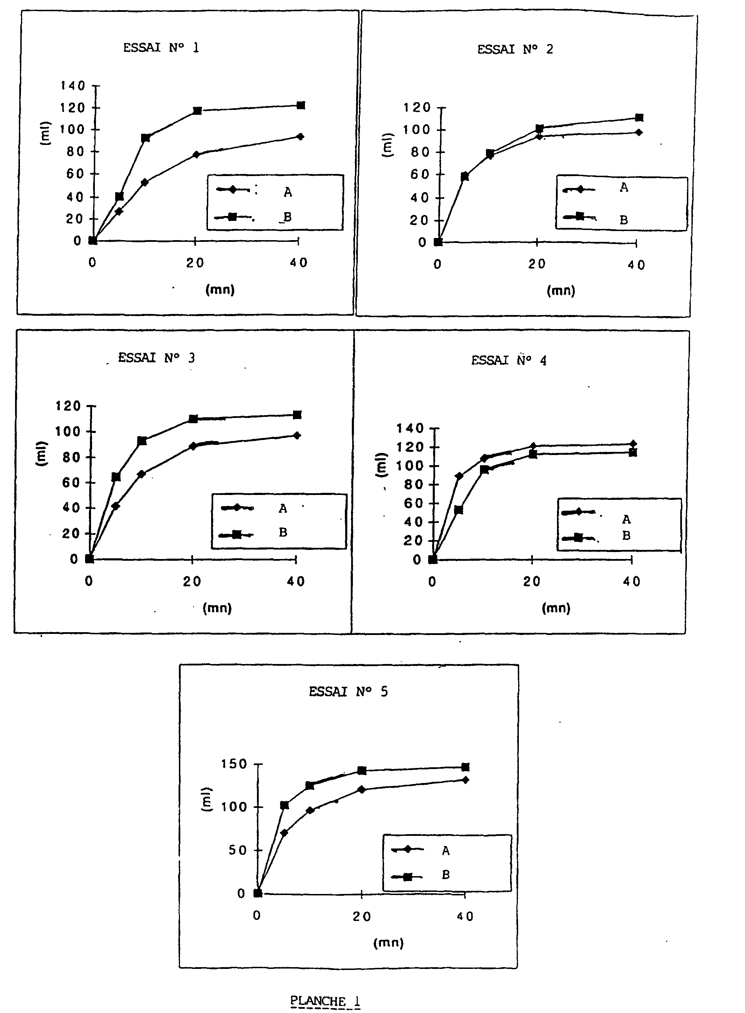

La planche 1/1 rassemble les résultats obtenus. Le symbole A définit les valeurs au premier jour de la fabrication - le symbole B définit les valeurs après 3 mois de conservation.

CONCLUSION

Après une période de conservation de 3 mois, les résultats de contrôle de dégagement de $CO_2$ montrent une augmentation très nette pour les formules 1, 3 et 5.

Ce phénomène s'explique par un retour à l'état stable des glycérides semi-synthétiques se traduisant par une diminution de la résistance à l'écrasement avec pour conséquence une meilleure réactivité entre les composants dans certaines formules.

Par contre, les formules 2 et 4 libèrent après conservation une quantité de $CO_2$ sensiblement la même

qu'après fabrication.

**Revendications**

1. Procédé d'obtention de compositions pharmaceutiques destinées à la voie rectale **caractérisé en ce que** l'on incorpore un sel à réaction acide choisi parmi le phosphate monosodique, le phosphate monopotassique, le citrate monosodique, l'acide pyroglutamique et l'acide glutamique et un bicarbonate de métal alcalin à une lécithine puis à un agent opacifiant et on introduit ce mélange dans un excipient gras tel qu'un stearate de glyceride semi-synthétique, fusible au voisinage de la température corporelle, pour former des suppositoires homogènes et de longue conservation, provoquant un dégagement gazeux maximal qui représente 80% du dégagement total dans les 5 premières minutes, au contact de l'ampoule rectale humide.

2. Procédé d'obtention de compositions pharmaceutiques selon la revendication 1 dans lequel la teneur en bicarbonate de sodium est d'environ 0,700 g et la teneur en citrate monosodique s'échelonne entre 1,25 g et 1,30 g par prise unitaire.

3. Procédé d'obtention de compositions pharmaceutiques selon la revendication 1 dans lequel la teneur en bicarbonate de sodium est d'environ 0,700 g et la teneur en acide pyroglutamique s'échelonne entre 0,85 g et 0,90 g par prise unitaire.

4. Procédé d'obtention de compositions pharmaceutiques selon la revendication 1 dans lequel la teneur en bicarbonate de sodium est d'environ 0,700 g et la teneur en phosphate monosodique s'échelonne entre 1,15 g et 1,20 g.

**Patentansprüche**

1. Verfahren zur Herstellung von pharmazeutischen Zusammensetzungen zur rektalen Verabreichung, **dadurch gekennzeichnet, dass** einem Lecithin und anschließend einem Trübungsmittel ein sauer reagierendes Salz ausgewählt aus der Gruppe umfassend Mononatriumphosphat, Monokaliumphosphat, Mononatriumcitrat, Pyroglutaminsäure und Glutaminsäure sowie ein alkalisches Metall-Bicarbonat beigemischt werden und dass diese Mischung in ein als Trägersubstanz dienendes, bei Körpertemperatur schmelzendes Fett, beispielsweise in ein halbsynthetisches Glyceridstearat, eingearbeitet wird, um so homogene, eine lange Haltbarkeit aufweisende Zäpfchen zu erzielen, die im feuchten Milieu der Ampulle in den ersten 5 Minuten eine maximale, 80 % der gesamten Gasmenge darstellende Gasmenge abgeben.

2. Verfahren zur Herstellung von pharmazeutischen Zusammensetzungen nach Anspruch 1, wobei in jedem einzelnen Zäpfchen etwa 0,700 g Natriumbicarbonat sowie zwischen 1,25 g und 1,30 g Mononatriumzitrat enthalten sind.

3. Verfahren zur Herstellung von pharmazeutischen Zusammensetzungen nach Anspruch 1, wobei in jedem einzelnen Zäpfchen etwa 0,700 g Natriumbicarbonat sowie zwischen 0,85 g und 0,90 g Pyroglutaminsäure enthalten sind.

4. Verfahren zur Herstellung von pharmazeutischen Zusammensetzungen nach Anspruch 1, wobei etwa 0,700 g Natriumbicarbonat sowie zwischen 1,15 g und 1,20 g Mononatriumphosphat enthalten sind.

**Claims**

1. A process for producing pharmaceutical compositions intended for the rectal way wherein a salt having an acidic reaction selected among monosodium phosphate, monopotassium phosphate, monosodium citrate, pyroglutamic acid and glutamic acid and an alkali metal bicarbonate is incorporated to a lecithin then to an opacifying agent and the whole mixture is introduced in a fatty excipient such as a semi synthetic glyceryl stearate which melts in the neighbourhood of the body temperature, to produce homogenous and with long shelf-life suppositories which insure a maximal gazeous evolvement representing 80% of the total evolvement in the five first minutes in this contact of the wet rectal ampul.

2. A process of producing pharmaceutical compositions according to claims 1, wherein the content in sodium bicarbonate is of about 0.700 g and the content in monosodium citrate ranges between 1,25 g and 1,70 g per unit dosage.

3. A process for producing pharmaceutical compositions according to claim 1, wherein the content in sodium bicarbonate is of about 0.700 g and the content in pyroglutamic acid ranges between 0.85 g and 0.90 g per unit dosage.

4. A process for producing pharmaceutical compositions according to claim 1, wherein the content in sodium bicarbonate is of about 0.700 g and the content is monosodium phosphate ranges between 1,15 g and 1,20 g.

PLANCHE 1